Europäisches Patentamt

European Patent Office

Office européen des brevets

(11) Veröffentlichungsnummer: **0 012 115**
**A1**

(12) **EUROPÄISCHE PATENTANMELDUNG**

(21) Anmeldenummer: 79810167.1

(22) Anmeldetag: 28.11.79

(51) Int. Cl.³: **A 61 K 33/30**
//(A61K33/30, 31/725),
(A61K33/30, 31/795)

(30) Priorität: 04.12.78 CH 12360/78

(43) Veröffentlichungstag der Anmeldung:
11.06.80 Patentblatt 80/12

(84) Benannte Vertragsstaaten:
BE CH DE FR GB NL

(71) Anmelder: **CIBA-GEIGY AG**
**Patentabteilung Postfach**
**CH-4002 Basel(CH)**

(72) Erfinder: **Lukas, Bohumir, Dr.**
**Laufenburgerstrasse 10/3**
**CH-4058 Basel(CH)**

(72) Erfinder: **Wiesendanger, Walter**
**Steingrubenweg 12**
**CH-4142 Münchenstein(CH)**

(72) Erfinder: **Schmidt-Ruppin, Karl Heinz, Dr.**
**Im Baumgarten 5**
**CH-4144 Arlesheim(CH)**

(54) Pharmazeutische Präparate zur topischen Behandlung von Virusinfektionen.

(57) Die Erfindung betrifft neue pharmazeutische Präparate zur topischen Behanlung von Infektionen durch Herpes-Viren, insbesondere solchen, die durch Herpes-Virus hominis verursacht wurden, z.B. von Herpes genitalis und Herpes dermatitis. Die Präparate enthalten als antivirale Wirkstoffe eine synergistische Kombination aus einem pharmazeutisch annehmbaren Salz eines sauren sulfatierten Polysaccharids oder sauren sulfatierten Polymeren und Zinkionen, und gegebenenfalls einen zusätzlichen Gehalt an Polyoxyäthylensorbitan-monolaurat und/oder -monooleat in üblichen topisch anwendbaren Arzneimittelformen, wie Cremes, Salben, Tinkturen, wässrigen Lösungen und vor allem Gelen.

EP 0 012 115 A1

4-11196/2/-


Neue pharmazeutische Präparate


Die Erfindung betrifft neue pharmazeutische
Präparate zur topischen Anwendung, insbesondere zur
topischen Behandlung von Virusinfektionen, die eine antiviral wirksame Kombination eines pharmazeutisch  annehmbaren Salzes eines sauren, sulfatierten Polysaccharids
oder sauren sulfatierten Polymeren und Zinkionen enthalten,
und die Verwendung dieser Präparate zur topischen Behandlung von Infektionen durch Herpes-Viren, insbesondere
solchen, die durch Herpes-Virus hominis (HVH) verursacht
wurden, z.B. von Herpes genitalis und Herpes dermatitis.


Es wurde überraschenderweise gefunden, dass
Salze von sauren sulfatierten Polysacchariden oder von
sauren sulfatierten Polymeren und Zinkionen zusammen
eine starke antivirale Wirkung ausüben, welche die Summe
der Wirkungen der Einzelkomponenten weit übertrifft. Die
synergistische Wirkung der beiden Komponenten lässt sich
in vitro, d.h. in Zellkulturen, z.B. auf Grund der Virus-
Inaktivierung, der Hemmung der Plaquebildung und der
Virus-Replikation nachweisen:


1. Inaktivierung von Herpes-Virus hominis
Die Präparate wurden in bidestilliertem Wasser in abgestuften Konzentrationen gemischt, mit HVH2/Ang. beimpft
und während 1 Stunde bei 35°C inkubiert. Die Bestimmung
des Virusgehaltes in den Kontakgemischen erfolgte auf
Hühnerembryofibroblasten im Plaquetest. Die Proben wurden
in Hanks'-Lösung + 0,05% Albumin verdünnt auf Zellmono-

- 2 -

layer von Hühnerembryofibroblasten gegeben. Nach 90 Minuten Virusabsorption wurden die Kulturen 2-mal gewaschen, mit 4 ml LY-Agaroverlay [Hanks'Lösung mit 0,5 % Lactalbuminhydrolysat und 0,01 % Yeastolate (Hefeextrakt)] versetzt und dann bis zur Zählung der Plaques (96 Stunden) bei 35°C inkubiert.

Tabelle Ia

| ZnSO$_4$ .7 H$_2$O | Präparate, mcg/ml : | | |
|---|---|---|---|
| | Heparin Natriumsalz * | | |
| | 0 | 33 | 100 |
| 0 | 5,10 [a] | 5,04 | 5,00 |
| 33 | 4,89 | 4,76 | 4,95 |
| 100 | 4,76 | 2,60 | 2,62 |
| 300 | 4,68 | < 1,00 | < 1,00 |

a) Virusgehalt: Die Werte bedeuten Log 10 der Anzahl der PFU/ml (Plaque forming Units/pro ml)

* 160 IE/mg (von Biofac AS, Kopenhagen, Dänemark)

Tabelle Ib

| Polysulfat-Salz | Konzentration in mcg/ml | $ZnSO_4 \cdot 7H_2O$ Konzentration in mcg/ml | | | |
|---|---|---|---|---|---|
| | | 0 | 11 | 33 | 100 |
| Keines | 0 | 6,06 a) | 5,33 | 4,98 | 4,65 |
| Kaliumsalz von Polyvinylsulfat Molekulargewicht ca. $8 \cdot 10^4$ | 11 33 100 | 5,34 5,27 4,78 | 3,20 2,95 2,74 | 2,84 2,39 2,30 | 2,50 2,27 2,17 |
| Natriumsalz von Dextransulfat Molekulargewicht ca. $5 \cdot 10^5$ | 11 33 100 | 5,39 5,22 4,61 | 4,72 4,05 3,14 | 4,63 3,32 2,39 | 3,74 2,69 2,54 |
| Natriumsalz von Dextransulfat Molekulargewicht ca. $2 \cdot 10^6$ | 11 33 100 | 5,49 5,30 4,67 | 4,54 4,05 3,43 | 4,30 3,62 2,95 | 3,69 3,02 2,90 |

a) vgl. Tabelle Ia.

2. **Hemmung der Plaquebildung von HVH2/Ang. in Hühnerembryofibroblasten.**

Die Zellmonolayer wurden mit 100 PFU HVH2/Ang. während 90 Minuten infiziert. Je 4 ml der in LY-Agaroverlay (mit 5 % Schafserum, 0,5 % OXO-L-28-Agar, ohne Diäthylaminoäthyldextran) inkorporierten Präparate wurden auf die infizierten Zellkulturen gegeben und diese bis zur Zählung der Plaques (96 Stunden) bei 35°C inkubiert.

<u>Tabelle IIa</u>

| Präparate, mcg/ml: ZnSO$_4$·7H$_2$O | Heparin Natriumsalz * | | | |
|---|---|---|---|---|
| | 0 | 1,5 | 3 | 6 |
| 0 | 100 )<br>2-3 ) | 82<br>2-3 | 87<br>2-3 | 76<br>1-2 |
| 3 | 104<br>1-3 | 65<br>1-2 | 57<br>0,5-1 | 42<br>0,5 |
| 6 | 64<br>1-2 | 31<br>0,5-1 | 11<br>0,5 | 2<br>0,5 |
| 12 | 43<br>0,5-1 | 8<br>0,5 | 2<br>0,5 | 2<br>0,5 |
| 25 | 15<br>0,5 | 5<br>0,5 | 2<br>0,5 | 1<br>0,5 |

a) Durchschnittliche Plaquezahl von 3 Schalen für jede
Konzentration in % der Plaquezahl des Kontrolltests
b) Plaquegrösse Ø  in mm (ca.)

* 160 IE/mg (Biofac)

- 5 -

Tabelle IIb

| Polysulfat-Salz | Konzentration in mcg/ml | $ZnSO_4 \cdot 7H_2O$ Konzentration in mcg/ml | | | | |
|---|---|---|---|---|---|---|
| | | O | 3 | 6 | 12 | 24 |
| Keines | O | 100[a] | 100 | 98 | 87 | 66 |
| Kaliumsalz von Polyvinylsulfat Molekulargewicht ca. $8 \cdot 10^4$ | 1,5 | 100 | 89 | 87 | 69 | 57 |
| | 3 | 88 | 75 | 59 | 48 | 9 |
| | 6 | 43 | 33 | 7 | 5 | 1 |
| Natriumsalz von Dextransulfat Molekulargewicht ca. $5 \cdot 10^5$ | 1,5 | 102 | 103 | 96 | 81 | 34 |
| | 3 | 75 | 84 | 77 | 61 | 9 |
| | 6 | 52 | 18 | 5 | 1 | O |
| Natriumsalz von Dextransulfat Molekulargewicht ca. $2 \cdot 10^6$ | 1,5 | 96 | 96 | 94 | 75 | 52 |
| | 3 | 92 | 100 | 76 | 61 | 32 |
| | 6 | 24 | 19 | 11 | 5 | 1 |

a) vgl. Tabelle IIa.Durchschnittliche Plaquezahlen von 6
Schalen für jede Konzentration. Mit der Anzahl der Plaques
nimmt auch deren Durchmesser von ca. 2-3 mm im Kontrolltest
auf $< 0,5$ mm in den Tests mit den wirksamsten Kombinationen
ab.

### 3. Hemmung der Replikation von HVH2/Ang. in VERO-Zellen bei präinfektiösem Behandlungsbeginn

Konfluente Monolayer von VERO-Zellen wurde mit F15-Medium [Minimum Essential Medium (Eagle), Gibco Bio-Cult Ldt. Paisley, Schottland] gewaschen und dann mit je 4 ml der verschiedenen Konzentrationen der Präparate in F15 Medium belegt. Die Infektion der Kulturen erfolgte nach 60 Minuten mit 1000 PFU HVH2/Ang. in 0,1 ml Medium. Nach 20 Stunden Inkubation bei 35°C wurde der Zustand der Zellen mikroskopisch beurteilt und der Virusgehalt der Zell-Lysate (Zellen mit 1 ml bidestilliertem Wasser 2-mal tiefgefroren und getaut) durch Titration auf Hühner-embryofibroblasten (Plaquebildung) bestimmt.

Tabelle III

| Präparate,mcg/ml :<br>$ZnSO_4.7H_2O$ | Heparin 0 | Natrium-salz * 6 |
|---|---|---|
| 0 | 4,58 [1] | 1,84 |
| 6 | 3,59 | $< 1,00$ |
| 12 | 3,32 | $< 1,00$ |
| 25 | 2,82 | $< 1,00$ |

1) Log10 PFU/ml Zell-Lysat
* 160 IE/mg (Biofac)

### Schlussfolgerungen aus den Resultaten der drei Versuchsreihen

In den obigen Versuchsanordnungen ist die synergistische Wirkung der erfindungsgemässen Kombinationen von Zinksulfat ($ZnSO_4 \cdot 7H_2O$) und Heparin-Natriumsalz entsprechend Tabellen Ia, IIa und III deutlich erkennbar. Besonders bemerkenswert ist bei der Virus-Inaktivierung gemäss Tabelle Ia, dass jede Komponente allein in allen geprüften Konzentrationen praktisch unwirksam war, während die Kombination von je einem Drittel der Maximalkonzentration der einzelnen Komponenten den Virusgehalt der Hühnerembryofibroblasten bereits um $10^2$ verminderte. Aus Tabelle IIa ergibt sich u.a., dass Heparin-Natriumsalz allein nur schwach wirksam ist, jedoch sowohl Heparin-Natriumsalz als auch Zinksulfat in der niedrigsten Konzentration zusammen mit jeder Konzentration der zweiten Komponente eine höhere Wirkung ergeben als die nächsthöhere Konzentration der zweiten Komponente allein. Mit der Kombination von 6 mcg/ml Zinksulfat und 3 mcg/ml Heparin-Natriumsalz wird bereits die Wirkung von 25 mgc/ml Zinksulfat allein, und mit je 6 mcg/ml Zinksulfat und Heparin-Natriumsalz wie auch mit 12 mcg/ml Zinksulfat und 3 mcg/ml Heparin-Natriumsalz nahezu die Wirkung der Kombination von 25 mcg/ml Zinksulfat und 6 mcg/ml Heparin-Natriumsalz erreicht. Die Virus-Replikation wird gemäss Tabelle III von Zinksulfat in der höchsten Konzentration von 25 mcg/ml nur mässig, dagegen von Heparin in der einzigen geprüften Konzentration von 6 mcg/ml, entsprechend der höchsten in den andern Versuchsreihen, infolge der starken Absorptionshemmwirkung des Heparin-Natriumsalz deutlich gehemmt; alle geprüften Kombinationen zeigen indessen eine noch mindestens 10-mal stärkere Wirkung als die Einzelkomponenten.

Aus den in den Tabellen Ib und IIb angegebenen Resultaten ist auch die synergistische Wirksamkeit der Kombinationen von Zinksulfat mit den Natriumsalzen von zwei Dextransulfaten von verschiedenen Molekulargewichten sowie mit dem Kaliumsalz von Polyvinylsulfat ersichtlich. Besonders bemerkenswert ist bei den Resultaten der Virus-Inaktivierung gemäss Tabelle Ib, dass die Komponenten für sich allein in der höchsten Konzentration von 100 mcg/ml den Virusgehalt um bloss ca. $10^{1,5}$ verminderten, d.h. gleich oder weniger stark als die Kombinationen von je einem Neuntel der Maximalkonzentrationen der Einzelkomponenten und dass demgegenüber die Kombinationen von je einem Drittel der Maximalkonzentration der Einzelkomponenten den Virusgehalt weit stärker, d.h. um ca. $10^{2,5}$ bis $10^{3,5}$ verminderten. Aus Tabelle IIb ist ersichtlich, dass die Wirkung der Polysulfat-Salze, welche für sich allein sogar in der höchsten Konzentration von 6 mcg/ml keinen starken Effekt zeigen, bedeutend gesteigert wird durch den Zusatz von Zinksulfat in den Konzentrationen von 6 mcg/ml und 12 mcg/ml, welche für sich allein unwirksam bzw. nahezu unwirksam sind.

4. <u>Wirksamkeit bei der durch Infektion mit HVH$_2$/Ang. verursachten Herpes genitalis des weiblichen Meerschwein-schens.</u>

 Ebenfalls festgestellt werden kann der synergistische Effekt der erfindungsgemäss kombinierten Wirkstoffe in vivo bei der durch Infektion mit HVH2/Ang. verursachten Herpes genitalis des weiblichen Meerschweinchens bei 72 Stunden nach intravaginaler Infektion (Stadium deutlicher Symptome) beginnender Behandlung, gemäss der von B. Lukas et al., Arch.Ges.Virusforsch.<u>44</u> , 153-155 (1974) und <u>49</u>, 1-11 (1975) beschriebenen Methodik. Die Resultate von entsprechenden Doppelblindversuchen sind in der nachstehenden Tabelle IV angegeben:

Tabelle IV

| No. | H IU | Z % | a) n | % Tiere mit mehr als 66% Regression am Tage | | | | % symptomfreie Tiere am Tage | | | | | Para- lyse % | Exitus % |
|---|---|---|---|---|---|---|---|---|---|---|---|---|---|---|
| | | | | 4 | 7 | 9 | 11 | 9 | 11 | 14 | 18 | 25 | | |
| 1 | - | -c) | 108 | 0 | 0 | 3 | 6 | 2 | 3 | 5 | 9 | 15 | 39 | 24 |
| 2 | - | -d) | 35 | 0 | 11 | 17 | 37 | 14 | 25 | 40 | 48 | 51 | 23 | 11 |
| 3 | 32 | - | 8 | 0 | 0 | 25 | 37 | 0 | 12 | 25 | 37 | 62 | 0 | 0 |
| 4 | 160 | - | 36 | 0 | 11 | 39 | 61 | 30 | 30 | 41 | 58 | 66 | 8 | 3 |
| 5 | - | 0,5 | 10 | 0 | 0 | 10 | 10 | 0 | 10 | 30 | 50 | 70 | 20 | 0 |
| 6 | - | 1,0 | 18 | 0 | 5 | 5 | 17 | 0 | 17 | 28 | 33 | 33 | 11 | 5 |
| 7 | 32 | 0,5 | 10 | 10 | 10 | 30 | 40 | 0 | 10 | 50 | 40 | 40 | 20 | 0 |
| 8 | 32 | 1,0 | 10 | 0 | 0 | 30 | 20 | 20 | 20 | 30 | 50 | 80 | 0 | 0 |
| 9 | 160 | 0,5 | 19 | 0 | 5 | 84 | 94 | 53 | 72 | 94 | 94 | 94 | 10 | 5 |
| 10 | 160 | 1,0 | 46 | 6 | 37 | 63 | 87 | 43 | 74 | 78 | 87 | 90 | 4 | 0 |

H = Heparin Natriumsalz, 160 IU/mg (Biofac)

Z = $ZnSO_4 \cdot 7H_2O$

a) Tierzahlen $> 10$ sind Gesamtzahlen der Versuchstiere von mehreren Versuchen mit je 8 oder 10 Tieren.

b) Tag O ist der Tag des Behandlungsbeginn 72 Stunden nach der Infektion . Die Behandlung besteht in der intravaginalen Applikation von 0,1 ml des zu prüfenden Präparats zweimal täglich während 5 aufeinanderfolgenden Tagen.

c) Infizierte Kontrolltiere, die keine Behandlung erhielten.

d) Infizierte Kontrolltiere, die mit der in allen Präparaten verwendeten Gel-Grundlage, die 0,1% Polyoxyäthylen-sorbitan-monooleat enthält, behandelt wurden.

Die Resultate der Vergleichsversuche zeigen, dass die Verabreichung von Heparin-Natriumsalz oder Zinkionen allein als Wirkstoff mit einer geeigneten Gel-Grundlage die günstige Wirkung der letzteren nur mässig verstärkt, da nach Behandlung mit einem der genannten Wirkstoffe allein am Ende der Versuchsperiode höchstens 70 % der Versuchstiere, indessen nach Behandlung mit der Gel-Grundlage allein bereits etwa 50 % der Versuchstiere symptomfrei sind. Im Gegensatz dazu führte die kombinierte Verabreichung von Zinkionen und Heparin-Natriumsalz im erfindungsgemässen Konzentrationsbereich zu einer beinahe vollständigen Heilung des experimentellen Krankheitszustandes, indem am Ende der Versuchsperiode 18 von 19 bzw. 42 von 46 Versuchstieren, d.h. mindestens 90 % symptomfrei waren. Ueberdies tritt auch das bei alleiniger Verabreichung von Heparin-Natriumsalz feststellbare frühe Einsetzen der kurativen Wirkung nach Verabreichung der Kombination noch erheblich stärker in Erscheinung, obwohl die Wirkung nach Verabreichung von Zinkionen allein erst spät einsetzt.

Die synergistische Wirkung der obengenannten Wirkstoffe wird noch verstärkt, wenn eine Präparategrundlage, insbesondere Gelgrundlage, verwendet wird, die einen oder mehrere Polyoxyäthylensorbitanfettsäureester, wie Polyoxyäthylensorbitan-monostearat und/oder vor allem Polyoxyäthylensorbitan-monolaurat oder in erster Linie Poloyoxyäthylensorbitan-monooleat, enthält.

Die erfindungsgemässen pharmazeutischen Präparate enthalten die oben definierten Wirkstoffe vorzugsweise in Kombination mit für die topische Applikation geeigneten pharmazeutischen Trägerstoffen. Als Formen von erfindungsgemässen Präparaten kommen insbesondere Tinkturen, Lösungen, Cremes, Salben und vor allem Gele in Betracht.

- 11 -

Tinkturen und Lösungen weisen meistens eine wässerig-äthanolische bzw. wässerige Grundlage auf, der u.a. Polyalkohole, wie z.B. Propylenglykol oder Glycerin und/oder niedrige Polyäthylenglykole, als Feuchthaltemittel zur Herabsetzung der Verdunstung, und gegebenenfalls rückfettende Substanzen, wie Fettsäureester von niedrigen Polyäthylenglykolen, d.h. im wässerig-äthanolischen Gemisch lösliche, lipophile Substanzen, als Ersatz für die der Haut mit dem Aethanol entzogenen Fettsubstanzen, und gegebenenfalls weitere Hilfs- und Zusatzstoffe, neben üblichen, wie den untengenannten, Konservierungs- mitteln z.B. auch die bereits erwähnten Polyoxyäthylen- sorbitan-fettsäureester, wie Polyoxyäthylensorbitan-monolaurat oder Polyoxyäthylensorbitanmonooleat, zugegeben sind.

Cremes sind Oel-in-Wasser-Emulsionen, die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristinat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B Fettsäureester von Polyalkoholen oder Aethylenoxidaddukte davon, wie Polyglycerinfettsäureester oder Polyoxyäthylen-sorbitan-fettsäureester (Tweens), ferner Polyoxyäthylenfettalkoholäther oder -fettsäureester, oder entsprechende ionische Emulgatoren, wie Alkalimetallsalze von Fettalkoholsulfaten, z.B. Natrium- laurylsulfat, Natriumcetylsulfat oder Natriumstearyl- sulfat, die man üblicherweise in Gegenwart von Fettalko- holen, z.B. Cetylakohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyäthylenglykole, ferner Konservierungsmittel, Riechstoffe, etc.

Salben sind Wasser-in-Oel-Emulsionen, die bis zu 70 %, vorzugsweise jedoch von etwa 20 % bis etwa 50 % Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole, oder Ester davon, z.B. Cetylalkohol oder Wollwachsalkohole, bzw. Wollwachs enthalten. Emulgatoren sind entsprechende lipophile Substanzen, wie Sorbitan-fettsäureester (Spans), z.B. Sorbitanoleat und/oder Sorbitaniososstearat. Zusätze zur Wasserphase sind u.a. Feuchthaltungsmittel, wie Polyalkohole, z.B. Glycerin, Propylenglykol, Sorbit und/oder Polyäthylenglykol, sowie Konservierungsmittel, Riechstoffe, etc.

Gele sind insbesondere wässrige Lösungen der Wirkstoffe, in denen Gelbildner, vorzugsweise solche aus der Gruppe der Celluloseäther, wie z.B. Methylcellulose, Hydroxyäthylcellulose oder Carboxymethylcellulose, oder der pflanzlichen Hydrokolloide, wie Natrium-alginat, Traganth oder Gummi arabicum, dispergiert und ausgequollen sind. Weiter enthalten die Gele vorzugsweise ebenfalls Feuchthaltemittel aus der Gruppe der Polyalkohole, wie Propylenglykol, Glycerin und/oder niedrige Polyäthylenglykole, sowie Netzmittel, z.B. Polyoxyäthylensorbitan-fettsäureester, wie Polyoxyäthylensorbitanmonostearat,-monolaurat oder -monooleat in Konzentrationen von ca. 0,02 bis 5 %. Als weitere Zusatzstoffe enthalten die Gele übliche Konservierungsmittel, z.B. Benzylakohol, Phenäthylalkohol, Phenoxyäthanol, p-Hydroxybenzoesäureniederalkylester wie der Methyl- und/oder Propylester, Sorbinsäure oder organische Quecksilberverbindungen, wie Merthiolat.

Die erfindungsgemässen Präparate können - neben den üblichen Konservierungsmitteln - auch weitere biologisch, z.B. antiphlogistisch oder antimikrobiell, wie antibakteriell, antifungal oder ebenfalls antiviral wirksame Stoffe, wie z.B. Flumethason, Neomycin, Gentamycin, Milchsäure oder Mikonazol enthalten. Die Herstellung der erfindungsgemässen Präparate erfolgt in an sich bekannter Weise.

Die vorliegende Erfindung betrifft insbesondere topisch anwendbare antivirale Präparate, die pharmazeutisch annehmbare Salze von sauren sulfatierten Polysacchariden oder sauren sulfatierten Polymeren, wie Heparin, und Zinkionen in einem Verhältnis von 1 mg zu 0,18 bis 18 mg, insbesondere von 1 mg zu 0,18 bis 4,5 mg, und gegebenenfalls Polyoxyäthylensorbitanmonolaurat und/oder -monooleat enthalten. Für Salze von Heparin beziehen sich obige Mengenangaben auf solche mit 160 IE/mg, von Salzen von anderem Heparin sind gleiche IE-Mengen einzusetzen. Die Zinkionen werden in Form der entsprechenden Mengen einer dissoziierbaren Zinkverbindung, z.B. von 0,8-80 mg bzw. 0,8-20 mg $ZnSO_4 \cdot 7H_2O$, zugefügt. Entsprechende topisch anwendbare Präparate, insbesondere Gele, ferner auch Tinkturen, wässrige Lösungen, Cremen oder Salben, enthalten beispielsweise pro g oder ml 0,1 bis 5 mg, insbesondere 0,25-3 mg eines pharmazeutisch annehmbaren Salzen eines sauren sulfatierten Polysacchorids oder sauren sulfatierten Polymeren, beispielsweise 16 bis 800 IE, insbesondere 40 bis 480 IE eines entsprechenden Salzes von Heparin, und 0,18 bis 18 mg Zinkionen, entsprechend z.B. ca. 0,8 bis 80 mg $ZnSO_4 \cdot 7H_2O$, und gegebenenfalls zusätzlich 0,2 bis 50 mg Polyoxyäthylensorbitan-monolaurat und/oder -monooleat. Besonders bevorzugt ist ein Gehalt von 80-320 IE eines pharmazeutisch

...annehmbaren Salzes von Heparin, 0,45 bis 4,5 mg Zinkionen und gegebenenfalls zusätzlich 0,5 bis 10 mg Polyoxyäthylen-sorbitan-monolaurat und/oder -monooleat pro g oder ml.

Anstelle eines pharmazeutisch annehmbaren Salzes von Heparin kann auch eine antiviral wirkungsgleiche Menge eines entsprechenden Salzes eines andern sauren sulfatierten Polysaccharids oder sauren sulfatierten Polymeren, wie z.B. einem der nachstehenden genannten, insbesondere von Dextransulfat bzw. Polyvinylsulfat, verwendet werden, wobei ein Gehalt, pro g oder ml, von 0,25-2 mg besonders bevorzugt ist.

Unter sauren sulfatierten Polysacchariden werden Polysaccharide verstanden, in denen einwertige Schwefelsäurereste $-SO_2-OH$ mit Sauerstoffatomen und/oder, falls, wie in Heparin vorhanden, Stickstoffatomen verbunden sind. Solche sauren sulfatierten Polysaccharide können natürlicher Herkunft sein, wie Heparin, Chondroitinsulfat (Chondroitinschwefelsäure) oder Karrageenin, oder durch Sulfatierung von natürlichen oder partiell abgebauten Polysacchariden hergestellt sein, wie sulfatierte Amylopektine, sulfatierte Dextrane, sulfatierte Polyglucosen oder sulfatierte Polypentosen. Diese Stoffe werden in der Form ihrer pharmazeutisch annehmbaren Salze, wie z.B. Kalium- und insbesondere Natriumsalze verwendet. Als solche seien das Natriumsalz von Heparin als übliche Handelsform des letzteren, weiter das Kalium- und das Magnesiumsalz von Heparin und die Natriumsalze von sulfatierten Dextranen von verschiedenen Molekulargewichten, z.B. $4,8 \cdot 10^4$, $5 \cdot 10^5$ oder $2 \cdot 10^6$ genannt. Saure sulfatierte Polymere sind Sulfatierungsprodukte von Hydroxygruppen enthaltenden Polymeren, wie z.B. saure sulfatierte Polyvinylalkohole (Polyvinylsulfate) von

verschiedenen Molekulargewichten, die wiederum in Form von

pharmazeutisch annehmbaren Salzen, wie den Natrium- oder vor allem Kaliumsalzen, verwendet werden. Allgemein sind als pharmazeutisch annehmbare Salze die Natrium- und Kaliumsalze von besonderer Bedeutung.

Die Zinkionen können, statt in Form von Zinksulfat, auch in Form einer andern dissozierbaren Zinkverbindung, wie z.B. Zinkchlorid, Zinkacetat oder Zinkcitrat, oder des Zinksalzes einer Säure oder eines andern Stoffes von saurem Charakter und eigenen biologischen, z.B. antibakteriellen oder antiphlogistischen, Eigenschaften, wie z.B. Zink-sudoxicam (Zinksalz des 4-Hydroxy-2-methyl-N-(2-thiazolyl)-1,2-benzothiazin-3-carboxamid-1,1-dioxid) zugefügt werden. Hierbei ist es nicht notwendig, dass die zugefügten Zink-salze in dem verwendeten Trägermedium vollständig dissoziert sind, sondern es genügt, wenn ein im Rahmen der weiter oben genannten Konzentrationsangaben liegender Gehalt an Zinkionen zusammen mit nicht-dissoziertem Salz vorliegt.

Die erfindungsgemässen Präparate eignen sich insbesondere zur Behandlung von Herpes genitalis, Herpes dermatitis und Herpes labialis. Zur Behandlung der beiden ersteren werden erfindungsgemässe Gele so frühzeitig als möglich, z.B. mittels Tube oder Applikator, 2-3 mal täglich, und zur Behandlung von Herpes labialis mehrmals täglich auf die erkrankten Körperstellen aufgetragen bis zum Abklingen der Symptome bzw. bis zur Abheilung. Erfindungsgemässe wässrige Lösungen können z.B. zum Spülen von erkrankten Körperhöhlen, insbesondere zur Behandlung von Herpes gingivostomatitis, oder zur Behandlung von Herpes keratokonjunktivitis verwendet werden.

Die nachfolgenden Beispiele beschreiben die Herstellung einiger typischer Applikationsformen; sie sollen jedoch den Umfang der Erfindung in keiner Weise beschränken.

Beispiel 1

Zur Herstellung von 10,0 Liter Gel vermischt man 200,0 g hochviskose Natrium-carboxymethylcellulose und 50,0 g Polyoxyäthylensorbitan-monostearat (TWEEN 60) mit 1000 g Glycerin und 6,5 Liter Aqua conservans und lässt das Gemisch zu einem homogenen Schleim ausquellen. Dann wird eine Lösung von $1,6 \cdot 10^6$ IE Heparin-Natriumsalz

(z.B. 10,0 g Heparin Biofac), 50,0 g Zinksulfat-heptahydrat und 10,0 g Polyoxyäthylensorbitan-monooleat (TWEEN 80) in 2.0 Liter Aqua conservans zugemischt. Schliesslich wird mit Aqua conservans auf 10,0 Liter ergänzt, sorgfältig gemischt und das erhaltene Gel in Tuben abgefüllt.

Unter Aqua conservans wird eine wässrige Lösung von 0,07 % p-Hydroxy-benzoesäure-methylester (Methylparaben) und 0,03 % p-Hydroxybenzoesäure-propylester (Propylparaben) verstanden. TWEEN 60 und TWEEN 80 sind geschützte Markenbezeichnungen der ICI of America Inc., Stamford, Connecticut 06904.

Anstelle von 50,0 g $ZnSO_4 \cdot 7H_2O$ kann man auch 100,0 g verwenden und im übrigen gleich vorgehen wie oben angegeben.

Beispiel 2

Analog Beispiel 1 wird ein Gel hergestellt unter Verwendung von 5,0 g Natriumsalz von Dextransulfat mit einem Molekulargewicht von etwa $4,8 \cdot 10^4$ anstelle des Heparins.

Beispiel 3

Analog Beispiel 1 wird ein Gel hergestellt unter Verwendung von 2,5 g Natriumsalz von Dextransulfat mit einem Molekulargewicht von etwa $2 \cdot 10^6$ anstelle des Heparins.

Beispiel 4

Analog Beispiel 1 wird ein Gel hergestellt unter Verwendung von 2,5 g Kaliumsalz von Polyvinylsulfat mit einem Molekulargewicht von etwa $8 \cdot 10^4$ anstelle des Heparins.


Beispiel 5

Zur Herstellung von 10,0 Liter Gel vermischt man 200,0 g hochvisköse Natrium-carboxymethylcellulose und 50,0 g Polyoxyäthylensorbitan-monostearat (TWEEN 60, vgl. Beispiel 1) mit 1000 g Glycerin und 6,5 Liter Aqua conservans und lässt das Gemisch zu einem homogenen Schleim ausquellen. Dann wird eine Lösung von 5,0 g Natriumsalz von Dextransulfat mit einem Molekulargewicht von etwa $5 \cdot 10^5$, 50,0 g Zinksulfatheptahydrat und 10,0 g Polyoxyäthylensorbitan-monooleat (TWEEN 80, vgl. Beispiel 1) in 2,0 Liter Aqua conservans (vgl. Beispiel 1) zugemischt. Schliesslich wird mit Aqua conservans auf 10,0 Liter ergänzt, sorgfältig gemischt und das erhaltene Gel in Tuben abgefüllt.

Patentansprüche

1.   Pharmazeutische Präparate zur topischen Anwendung, gekennzeichnet durch einen Gehalt an einer antiviral wirksamen Kombination eines pharmazeutisch annehmbaren Salzes eines sauren, sulfatierten Polysaccharids oder sauren sulfatierten Polymeren und Zinkionen.

2.   Pharmazeutische Präparate gemäss Anspruch 1 zur topischen Behandlung von Virusinfektionen.

3.   Pharmazeutische Präparate gemäss Anspruch 1 in der Form von Gelen, Cremes, Salben, Tinkturen oder wässerigen Lösungen.

4.   Pharmazeutische Präparate gemäss Anspruch 1, gekennzeichnet durch einen zusätzlichen Gehalt an Polyoxyäthylensorbitan-monolaurat und/oder -monooleat.

5.   Pharmazeutische Präparate gemäss Anspruch 1, gekennzeichnet durch einen Gehalt an pharmazeutisch annehmbaren Salzen von sauren sulfatierten Polysacchariden oder sauren sulfatierten Polymeren und Zinkionen in einem Mengenverhältnis von 1 mg zu 0,18 bis 18 mg.

6.   Pharmazeutische Präparate gemäss Anspruch 5, gekennzeichnet durch einen Gehalt an pharmazeutisch annehmbaren Salzen von sauren sulfatierten Polysacchariden oder sauren sulfatierten Polymeren und Zinkionen in einem Mengenverhältnis von 1 mg zu 0,18 bis 4,5 mg.

7.   Pharmazeutische Präparate gemäss Anspruch 5, gekennzeichnet durch einen Gehalt an einem pharmazeutisch

annehmbaren Salz von Heparin und Zinkionen in einem Verhältnis von 160 IE zu 0,18 bis 18 mg.

8. Pharmazeutische Präparate gemäss Anspruch 5, gekennzeichnet durch einen Gehalt, pro g oder ml, von 0,1 bis 5 mg eines pharmazeutisch annehmbaren Salzes eines sauren sulfatierten Polysaccharids oder sauren sulfatierten Polymeren und 0,18 bis 18 mg Zinkionen.

9. Pharmazeutische Präparate gemäss Anspruch 5, gekennzeichnet durch einen Gehalt, pro g oder ml, von 0,25 bis 3 mg eines pharmazeutisch annehmbaren Salzes eines sauren sulfatierten Polysaccharids oder sauren sulfatierten Polymeren und 0,18 bis 18 mg Zinkionen.

10. Pharmazeutische Präparate gemäss Anspruch 5, gekennzeichnet durch einen Gehalt, pro g oder ml, von 0,16 bis 800 IE eines pharmazeutisch annehmbaren Salzes von Heparin und 0,18 bis 18 mg Zinkionen.

11. Pharmazeutische Präparate gemäss Anspruch 5, gekennzeichnet durch einen Gehalt, pro g oder ml, von 40 bis 480 IE eines pharmazeutisch annehmbaren Salzes von Heparin und 0,18 bis 18 mg Zinkionen.

12. Pharmazeutische Präparate gemäss Anspruch 5, gekennzeichnet durch einen Gehalt, pro g oder ml, von 80 bis 320 IE eines pharmazeutisch annehmbaren Salzes von Heparin und 0,45 bis 4,5 mg Zinkionen.

13. Pharmazeutische Präparate gemäss einem der Ansprüche 5, 7, 10, 11 und 12, die als pharmazeutisch annehmbares Salz von Heparin das Natriumsalz enthalten.

14. Pharmazeutische Präparate gemäss Anspruch 5, gekennzeichnet durch einen Gehalt, pro g oder ml, von 0,1-5 mg eines pharmazeutisch annehmbaren Salzes von Dextransulfat. und 0,18 bis 18 mg Zinkionen.

15. Pharmazeutische Präparate gemäss Anspruch 5, gekennzeichnet durch einen Gehalt, pro g oder ml, von 0,25-3 mg eines pharmazeutisch annehmbaren Salzes von Dextransulfat und 0,18 bis 18 mg Zinkionen.

16. Pharmazeutische Präparate gemäss Anspruch 5, gekennzeichnet durch einen Gehalt, pro g oder ml, von 0,25-2 mg eines pharmazeutisch annehmbaren Salzes von Dextransulfat und 0,45 bis 4,5 mg Zinkionen.

17. Pharmazeutische Präparate gemäss Anspruch 5, gekennzeichnet durch einen Gehalt, pro g oder ml, von 0,1-5 mg eines pharmazeutisch annehmbaren Salzes von Polyvinylsulfat und 0,18 bis 18 mg Zinkionen.

18. Pharmazeutische Präparate gemäss Anspruch 5, gekennzeichnet durch einen Gehalt, pro g oder ml, von 0,25-3 mg eines pharmazeutisch annehmbaren Salzes von Polyvinylsulfat und 0,18 bis 18 mg Zinkionen.

19. Pharmazeutische Präparate gemäss Anspruch 5, gekennzeichnet durch einen Gehalt, pro g oder ml, von 0,25-2 mg eines pharmazeutisch annehmbaren Salzes von Polyvinylsulfat. und 0,45 bis 4,5 mg Zinkionen.

20. Pharmazeutische Präparate gemäss einem der Ansprüche

1 bis 12 und 14 bis 19, dadurch gekennzeichnet, dass sie als pharmazeutisch annehmbares Salz eines sauren sulfatierten Polysaccharids oder sauren sulfatierten Polymers ein Natrium- oder Kaliumsalz enthalten.

21.     Pharmazeutische Präparate gemäss Ansprüchen 5 bis 20, denen die Zinkionen in Form von $ZnSO_4 \cdot 7H_2O$ als dissozierbare Zinkverbindung zugefügt sind.

22.     Pharmazeutische Präparate gemäss Ansprüchen 5 bis 20, gekennzeichnet durch einen zusätzlichen Gehalt, pro g oder ml, von 0,2 bis 50 mg Polyoxyäthylensorbitan-monolaurat und/oder -monooleat.

23.     Pharmazeutische Präparate gemäss Ansprüchen 5 bis 20, gekennzeichnet durch einen zusätzlichen Gehalt, pro g oder ml, von 0,5 bis 10 mg Polyoxyäthylensorbitan-monolaurat und/oder -monooleat.

24.     Verwendung der Präparate gemäss Ansprüchen 1 bis 23 zur topischen Behandlung von Infektionen durch Herpes-Viren.

25.     Verwendung der Präparate gemäss Ansprüchen 1 bis 23 zur topischen Behandlung von Herpes genitalis.

26.     Verwendung der Präparate gemäss Ansprüchen 1 bis 23 zur topischen Behandlung von Infektionen durch Herpes dermatitis.

**EUROPÄISCHER TEILRECHERCHENBERICHT,**
der nach Regel 45 des Europäischen Patentübereinkommens für das weitere Verfahren als
europäischer Recherchenbericht gilt

Europäisches
Patentamt

Nummer der Anmeldung 0012115

EP 79 81 0167

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| E | EP - A - 0 000 133 (CIBA-GEIGY AG)<br><br>* Seiten 12,13; Ansprüche 1-13 *<br><br>-- | 1-23 |
| A | DE - A - 2 715 711 (ROBUGEN GmbH PHARMAZEUTISCHE FABRIK)<br><br>* Seite 1, Ansprüche 1-4 *<br><br>-- | 1 |
| A | CHEMICAL ABSTRACTS, Band 66, Nr. 11, 13. März 1967, Zusammenfassung Nr. 45191s, Seite 4277, Columbus, Ohio, US, F. LEHEL et al.: "Effect of heparin on Herpes simplex virus infection in the rabbit"<br><br>& Acta Microbiol.Acad.Sci.Hung. 13(3), 197-203 (1966)<br><br>* Zusammenfassung *<br><br>---- | 1 |

**KLASSIFIKATION DER ANMELDUNG (Int Cl.³)**

A 61 K 33/30 //
(A 61 K 33/30
        31/725)
(A 61 K 33/30
        31/795)

**RECHERCHIERTE SACHGEBIETE (Int Cl.³)**

A 61 K 33/30

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:

Unvollständig recherchierte Patentansprüche:

Nicht recherchierte Patentansprüche: 24-26 Verfahren zur chirurgischen oder therapeutischen Behandlung des menschlichen oder tierischen Körpers. (Siehe Art. 52(4) des europäischen Patentübereinkommens).

Grund für die Beschränkung der Recherche:

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

| Recherchenort | Bdatum der Recherche | Prüfer |
|---|---|---|
| Den Haag | 14-03-1980 | BRINKMANN |

EPA Form 1505.1   06.78